# EUROPÄISCHE PATENTSCHRIFT

(11) **EP 2 422 737 B1**
(45) Veröffentlichungstag und Bekanntmachung des Hinweises auf die Patenterteilung: **02.10.2013**
(21) Anmeldenummer: 10173768.2
(22) Anmeldetag: 24.08.2010
(51) Int. Cl.: A61C 3/02, A61C 19/06, A61B 17/16, A61C 1/00

(54) **Vorrichtung für den Schnellstopp eines medizinischen, insbesondere dentalen, Bohrwerkzeugs**
Device for the immediate stop of a medical, in particular dental, drilling tool
Dispositif pour l'arrêt rapide d'un outil de forage médical, notamment dentaire

(43) Veröffentlichungstag der Anmeldung: 29.02.2012
(73) Patentinhaber: W & H Dentalwerk Bürmoos GmbH, 5111 Bürmoos (AT)
(72) Erfinder: Pruckner, Christian, 1190, Wien (AT); Jindra, Thomas, 5111, Bürmoos (AT); Watzek, Georg, Prof. Dr., 1030, Wien (AT); Unger, Ewald, 1210, Wien (AT)
(74) Vertreter: Benda, Ralf

(56) Entgegenhaltungen:
- EP-A2- 1 269 933
- WO-A2-2009/138242
- US-A1- 2009 245 956

## Beschreibung

Die vorliegende Erfindung betrifft eine Vorrichtung für den Schnellstopp eines medizinischen, insbesondere dentalen, Bohrwerkzeugs gemäß dem Oberbegriff des Anspruchs 1.

Eine derartige Vorrichtung ist zum Beispiel aus den Patentanmeldungen DE 10 2008 032 704 A1 oder EP 1 269 933 bekannt. Die Vorrichtung umfasst ein Bohrwerkzeug mit zwei konzentrischen, axial gegeneinander verschiebbaren Teilen, wobei das Innenteil gegenüber dem Außenteil axial in Bohrrichtung mittels einer Feder vorgespannt ist und ohne oder bei zu geringer Kraftgegenbeaufschlagung mit seiner Spitze gegenüber der Spitze des Außenteils in Bohrrichtung etwas vorsteht. Es ist des Weiteren eine Einrichtung vorgesehen, die bei einer axialen Verschiebung des Innenteils gegenüber dem Außenteil den Antrieb des Bohrwerkzeugs unterbricht, wobei die Einrichtung mit einem elektromagnetischen Sensor verbunden ist, der die axiale Verschiebung des Innenteils zum Außenteil detektiert.

Der Sensor ist als Endlagensensor ausgebildet, der an dem rückwärtigen (den Anschluss an einen Antrieb aufweisenden) Ende des Bohrwerkzeugs angeordnet ist und eine Bewegung des verbreiterten Endes des Innenteils, das aus dem Außenteil des Bohrwerkzeugs heraussteht, detektiert. Dieser Aufbau ist jedoch von erheblichem Nachteil wenn die Vorrichtung für den Schnellstopp in einen Handstückkopf, insbesondere in einen Winkelstückkopf, integriert werden soll, da insbesondere aufgrund der beengten Platzverhältnisse im Handstückkopf kaum Raum für den Sensor und für den Stellweg des verbreiterten Endes des Innenteils vorhanden ist. Zusätzlich behindert der Endlagensensor den Anschluss des Bohrwerkzeugs an eine Fluidversorgung.

Der vorliegenden Erfindung liegt daher die Aufgabe zugrunde eine Vorrichtung für den Schnellstopp eines medizinischen, insbesondere dentalen, Bohrwerkzeugs zu schaffen, welche die im Vorstehenden genannten Nachteile nicht aufweist und welche insbesondere derart ausgebildet ist, dass sie den beengten Platzverhältnisse in einem Handstückkopf, insbesondere in einem Winkelstückkopf, Rechnung trägt.

Diese Aufgabe wird gemäß einem Ausführungsbeispiel durch eine Vorrichtung für den Schnellstopp eines medizinischen, insbesondere dentalen, Bohrwerkzeugs gelöst, die aufweist: Ein Bohrwerkzeug mit einem Anschlussende zur Verbindung mit einem Antrieb, einem abrasiven Arbeitsende zum Materialabtrag und einem sich zwischen dem Anschlussende und dem Arbeitsende erstreckenden Körper mit einer sich entlang einer Längsachse des Bohrwerkzeugs erstreckenden Längsausdehnung, wobei das Bohrwerkzeug eine hohle Außenhülse aufweist, in welcher ein durch ein Federelement vorgespannter Fühler aufgenommen ist, der relativ zur Außenhülse entlang der Längsachse bewegbar ist, so dass zumindest ein Teil des Fühlers durch eine Öffnung der Außenhülse am Arbeitsende aus der Außenhülse bewegbar ist, wobei der Fühler als länglicher, sich entlang der Längsachse erstreckender Stift mit einem ersten, dem Anschlussende zugewandten Ende und einem zweiten, dem Arbeitsende zugewandten Ende ausgebildet ist, und einen elektromagnetischen Sensor zur Detektion einer Relativbewegung zwischen der Außenhülse und dem Fühler, wobei das erste, dem Anschlussende zugewandte Ende des Fühlers im Inneren der hohlen Außenhülse des Bohrwerkzeugs aufgenommen ist, der elektromagnetische Sensor entlang der Längsausdehnung des Körpers des Bohrwerkzeugs angeordnet ist und das Bohrwerkzeug einen sich entlang der Längsachse erstreckenden Leitungskanal für ein Behandlungsfluid aufweist.

Durch die Anordnung des elektromagnetischen Sensors entlang der Längsausdehnung des Körpers des Bohrwerkzeugs und des ersten, dem Anschlussende zugewandte Ende des Fühlers im Inneren der hohlen Außenhülse des Bohrwerkzeugs wird im Handstück, am rückwärtigen Ende (Anschlussende) des Bohrwerkzeugs kein zusätzlicher Patz für den Sensor benötigt. Somit besteht des Weiteren die Möglichkeit das Bohrwerkzeug an dessen Anschlussende mit einer Fluidquelle für ein Behandlungsfluid und / oder Kühlfluid zu verbinden und das Behandlungsfluid durch das Bohrwerkzeug entlang eines sich der Längsachse erstreckenden Leitungskanal zu leiten.

Als elektromagnetischer Sensor im Sinne der vorliegenden Schrift werden sowohl Sensoren verstanden, die primär oder ausschließlich auf elektrische Felder reagieren, zum Beispiel kapazitive Sensoren, als auch Sensoren, die primär oder ausschließlich auf magnetische Felder reagieren, zum Beispiel induktive Sensoren oder Magnetsensoren.

Gemäß einem Ausführungsbeispiel weist der elektromagnetische Sensor einen induktiven Sensor mit zumindest einer Spule und einem Spulenkern, zum Beispiel einem hart- oder weichmagnetischen Magnetelement, insbesondere einem Ferritkörper, auf, wobei die zumindest eine Spule und der Spulenkern relativ zueinander bewegbar sind, insbesondere durch die Relativbewegung des Fühlers zur Außenhülse. Bevorzugt ist der Spulenkern mit dem Fühler entlang der Längsachse des Bohrwerkzeugs und relativ zur zumindest einen Spule bewegbar. Unter einem induktiven Sensor wird erfindungsgemäß sowohl ein Sensor verstanden, der aufgrund der im Vorstehenden genannten Relativbewegung eine Induktivitätsänderung in der Spule detektiert (wobei dieser Sensor ein weichmagnetisches Magnetelement aufweist), als auch ein Sensor, welcher aufgrund der im Vorstehenden genannten Relativbewegung eine in der Spule erzeugte Induktionsspannung detektiert (wobei dieser Sensor ein permanentmagnetisches Magnetelement aufweist).

Gemäß einem Ausführungsbeispiel weist der elektromagnetische Sensor einen Magnetsensor, insbesondere einen Hall-Sensor oder einen Reed-Sensor, und zumindest ein Magnetelement auf, wobei der Magnetsensor und das zumindest eine Magnetelement durch die Relativbewegung des Fühlers zur Außenhülse relativ zueinander bewegbar sind.

Gemäß einem Ausführungsbeispiel weist der elektromagnetische Sensor einen kapazitiven Sensor mit zumindest zwei metallischen Elektroden auf, die einen Kondensator bilden, wobei eine Elektrode durch die Relativbewegung des Fühlers zur Außenhülse relativ zu einer anderen Elektrode bewegbar ist. Bevorzugt umfasst der kapazitive Sensor zumindest zwei im Wesentlichen plattenförmige Elektroden sowie eine mit dem Fühler entlang der Längsachse des Bohrwerkzeugs und relativ zu den zwei im Wesentlichen plattenförmigen Elektroden bewegbare Messelektrode.

Die vorgenannten Ausführungsbeispiele, insbesondere die verwendeten Sensoren, haben mehrere Vorteile: Die Sensoren sind als berührungslose Sensoren ausgebildet und arbeiten im Wesentlichen verschleißfrei. Die Abmessungen der Sensoren sind gering, so dass zumindest Teile der Sensoren im Bohrwerkzeug integriert werden können und / oder am oder in der Nähe des Bohrwerkzeugs angebracht werden können, ohne die Verwendung des Bohrwerkzeugs zu stören oder zu beeinflussen. Die Sensoren oder zumindest Teile davon sind gegenüber äußeren Einflüssen, wie zum Beispiel Behandlungsfluiden, Flüssigkeiten, Dämpfen, Reinigungsmitteln, Partikeln, resistent oder zumindest Teile der Sensoren sind in einer Kapselung integrierbar, um gegenüber den äußeren Einflüssen resistent zu sein. Insbesondere können zumindest Teile der Sensoren, zum Beispiel ein Magnetelement, ein Ferritkörper oder eine Platte/Elektrode des Kondensators derart verkleinert werden, dass sie in der hohlen Außenhülse / im Inneren der hohlen Außenhülse des Bohrwerkzeugs anordenbar sind, besonders bevorzugt mit dem Fühler verbunden, und trotz ihrer Verkleinerung überraschender Weise ein ausreichend starkes, auswertbares oder verarbeitbares Sensorsignal erzeugen. Der Durchmesser oder die Breite eines mit dem Fühler verbundenen Sensorelements, zum Beispiel eines Magnetelements, eines Ferritkörpers oder einer Platte/Elektrode des Kondensators, beträgt bevorzugt weniger als 3,0 mm, besonders bevorzugt weniger als 2,5 mm.

Gemäß einem Ausführungsbeispiel weist der Leitungskanal für ein Behandlungsfluid eine Bohrung in der Außenhülse des Bohrwerkzeugs auf und / oder durchsetzt das den Fühler vorspannende Federelement. Gemäß einem anderen Ausführungsbeispiel weist der Leitungskanal für ein Behandlungsfluid eine Bohrung in dem Fühler des Bohrwerkzeugs auf. Bevorzugt weist der Fühler einen Führungsabschnitt, dessen Durchmesser in etwa der lichten Weite der hohlen Außenhülse, in welcher er aufgenommen ist, entspricht, so dass der Führungsabschnitt an der Innenwand der hohlen Außenhülse gelagert ist, und einen zweiten Abschnitt auf, der durch einen Spalt, insbesondere einen Ringspalt, von der Innenwand der hohlen Außenhülse beabstandet ist, wobei der Spalt zumindest einen Teil des Leitungskanals für ein Behandlungsfluid bildet. Besonders bevorzugt sind die Bohrung in dem Fühler, die den Leitungskanal für ein Behandlungsfluid bildet, und der Spalt durch eine Querbohrung in dem Fühler miteinander verbunden.

Diese Ausführungsbeispiele haben den Vorteil, dass trotz der Integration zumindest eines Teils des Sensors in das Bohrwerkzeug eine Leitung des Behandlungsfluids durch das Innere des Bohrwerkzeugs und eine Abgabe des Behandlungsfluids direkt an der Spitze oder am abrasiven Arbeitsende des Bohrwerkzeugs und damit eine besonders effektive Kühlung des Bohrwerkzeugs und der Behandlungsstelle unmittelbar dort, wo das abrasive Arbeitsende des Bohrwerkzeugs am Knochen angreift, realisierbar ist.

Gemäß einem Ausführungsbeispiel weist der Fühler an seinem zweiten, dem Arbeitsende zugewandten Ende eine exzentrisch zur Längsachse angeordnete Fühlerspitze auf. Dies ist insbesondere von Vorteil wenn das Bohrwerkzeug schief auf das zu durchbohrende Material aufgesetzt ist oder die Dicke des zu durchbohrenden Materials variiert, da durch die rotierende, exzentrisch angeordnete Fühlerspitze eine größere Fläche des angebohrten Materials abgetastet wird und somit der dünnste Bereich des Materials besser erkannt werden kann. Die Fühlerspitze kann des Weiteren mit einer Schneide oder einer abrasiven Oberfläche zum Abtragen von Gewebe versehen sein.

Die exzentrisch zur Längsachse angeordnete Fühlerspitze ermöglicht auch eine Drehmitnahme der Fühlerspitze durch die hohle Außenhülse des Bohrwerkzeugs, wobei bevorzugt die exzentrische Fühlerspitze eine zur Längsachse gewandte Mitnehmerfläche aufweist, die eine, insbesondere im Wesentlichen zentrische, Mitnehmerfläche der hohlen Außenhülse kontaktiert, so dass die Antriebsbewegung der hohlen Außenhülse auf den Fühler übertragbar ist und der Fühler sich gemeinsam mit der hohlen Außenhülse dreht.

Eine medizinische, insbesondere dentale, Behandlungsvorrichtung umfasst gemäß einem Ausführungsbeispiel eine erfindungsgemäße Vorrichtung für den Schnellstopp eines medizinischen, insbesondere dentalen, Bohrwerkzeugs, einen Antrieb für das Bohrwerkzeug, ein Handstück zum Anschluss des Bohrwerkzeugs und eine Steuer- und / oder Regelvorrichtung zum Empfang eines von dem elektromagnetischen Sensor erzeugten Sensorsignals und zum Stoppen des Antriebs, wobei zumindest ein Teil des elektromagnetischen Sensors am Handstück angeordnet ist, wobei insbesondere ein Teil des elektromagnetischen Sensors direkt am Handstück befestigt oder befestigbar ist und damit nicht direkt am Bohrwerkzeug befestigt ist. In anderen Worten besteht der Sensor somit aus zwei Teilen, wovon ein Teil fest oder lösbar direkt mit dem Handstück verbunden ist und ein zweiter Teil direkt mit dem Bohrwerkzeug verbunden ist, so dass, wenn das Handstück und das Bohrwerkzeug voneinander getrennt werden, jeweils ein Teil des Sensors am Handstück und ein Teil des Sensors am Bohrwerkzeug verbleibt.

Gemäß einem Ausführungsbeispiel ist der Sensor im Bereich der Werkzeug-Einstecköffnung des Handstücks vorgesehen. Dies ist insbesondere von Vorteil, wenn das Bohrwerkzeug einen Abschnitt mit einem größeren Durchmesser (im Vergleich zum Durchmesser eines anderen Abschnitts des Bohrwerkzeugs) aufweist, zum Beispiel wenn jener Abschnitt einen größeren Durchmesser hat, in oder an dem zumindest ein Teil des Sensors vorgesehen ist. Insbesondere kann die Werkzeug-Einstecköffnung einen Abschnitt mit einem vergrößerten Durchmesser aufweisen, an dem der direkt mit dem Handstück verbundene Teil des Sensors vorgesehen ist und in den der Abschnitt des Bohrwerkzeugs mit dem größeren Durchmesser aufnehmbar ist. Auf diese Weise ist der Sensor am Handstück angeordnet, ohne dass andere Bauteile des Handstücks oder deren Anordnung im Handstück beeinflusst oder geändert werden müssten oder dass andere Bauteile des Handstücks den Sensor oder die Funktion oder den Betrieb des Sensors beeinflussen.

Gemäß einem bevorzugten Ausführungsbeispiel ist ein erster Teil des Sensors am Bohrwerkzeug vorgesehen und durch den Anschluss des Bohrwerkzeugs an das Handstück lösbar mit dem Handstück verbindbar und ist ein zweiter Teil des Sensors über eine lösbare Verbindungsvorrichtung direkt am Handstück befestigbar, insbesondere an der Außenhülse des Handstücks, so dass, wenn der erste und der zweite Teil des Sensors am Handstück befestigt sind, der erste und der zweite Teil des Sensors operativ miteinander in Wechselwirkung treten. Damit ist der Sensor in vorteilhafter Weise vollständig von dem Handstück lösbar und zum Beispiel an unterschiedliche Handstücke anschließbar oder kann separat von dem Handstück gereinigt oder gepflegt werden. Besonders bevorzugt sind der erste Teil des Sensors und / oder der zweite Teil des Sensors an der Außenseite der Außenhülse und / oder außerhalb der Außenhülse des Handstücks anordenbar. Damit kann ein beliebiges Handstück mit dem Sensor oder der Vorrichtung für den Schnellstopp verbunden werden und mit einem beliebigen Handstück ein Bohrer-Schnellstopp durchgeführt werden, ohne dass das Handstück adaptiert werden muss.

Die Erfindung wird nachfolgend anhand bevorzugter Ausführungsbeispiele und Bezug nehmend auf die beigefügten Zeichnungen erläutert:
Figur 1 zeigt eine perspektivische Darstellung eines Ausführungsbeispiels eines medizinischen, insbesondere dentalen, Bohrwerkzeugs einer Vorrichtung für den Schnellstopp.
Die Figuren 2A und 2B zeigen Schnittdarstellungen durch ein Ausführungsbeispiel einer Vorrichtung für den Schnellstopp eines medizinischen, insbesondere dentalen, Bohrwerkzeugs mit einem induktiven Sensor, wobei die Schnittebenen der beiden Figuren 90° zueinander gedreht sind.
Die Figur 3A zeigt eine Außenansicht eines Ausführungsbeispiels einer Vorrichtung für den Schnellstopp eines medizinischen, insbesondere dentalen, Bohrwerkzeugs, wobei das Bohrwerkzeug auf eine Oberfläche aufgesetzt ist, so dass die Fühlerspitze des Fühlers entgegen der Kraft des Federelements in die Außenhülse des Bohrwerkzeugs eingeschoben ist.
Die Figuren 3B und 3C zeigen Schnittdarstellungen durch die Vorrichtung für den Schnellstopp der Figur 3A, wobei die Schnittebenen der beiden Figuren 90° zueinander gedreht sind.
Figur 4 zeigt eine Schnittdarstellung durch ein Ausführungsbeispiel einer Vorrichtung für den Schnellstopp eines medizinischen, insbesondere dentalen, Bohrwerkzeugs mit einem kapazitiven Sensor.
Figur 5 zeigt eine Schnittdarstellung durch ein Ausführungsbeispiel einer Vorrichtung für den Schnellstopp eines medizinischen, insbesondere dentalen, Bohrwerkzeugs mit einem Magnetsensor.
Figur 6 zeigt eine teilweise schematische Darstellung einer Ausführungsform einer medizinischen, insbesondere dentalen, Behandlungsvorrichtung, mit einer Vorrichtung für den Schnellstopp eines Bohrwerkzeugs, einem Antrieb für das Bohrwerkzeug, einem Handstück zum Anschluss des Bohrwerkzeugs und einer Steuer- und / oder Regelvorrichtung zum Empfang eines von dem elektromagnetischen Sensor erzeugten Sensorsignals und zum Stoppen des Antriebs.
Die Figuren 7A, 7B zeigen eine medizinische, insbesondere dentale, Behandlungsvorrichtung, mit einer lösbar mit einem Handstück verbundenen Vorrichtung für den Schnellstopp eines medizinischen, insbesondere dentalen, Bohrwerkzeugs.

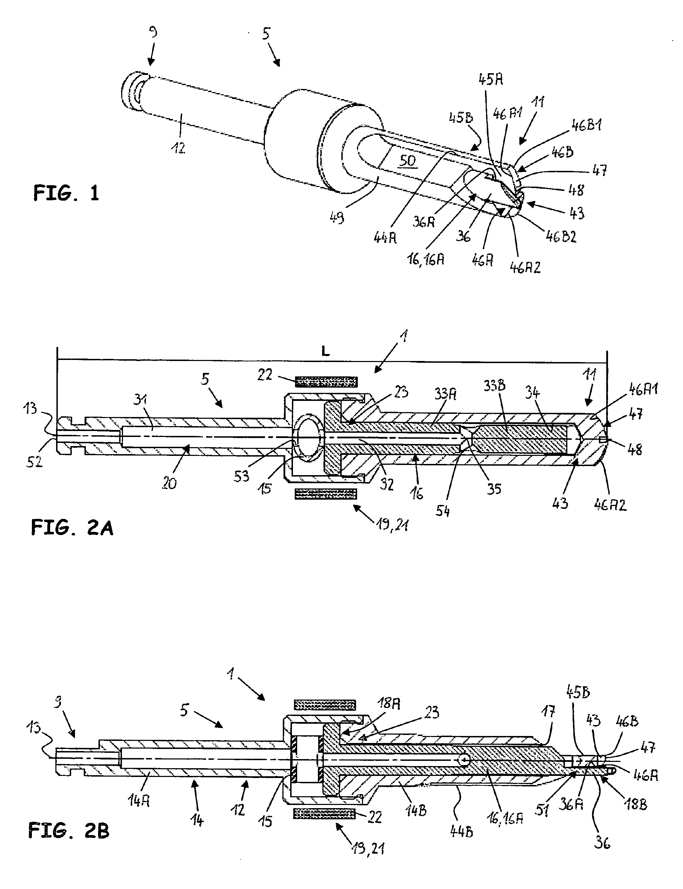

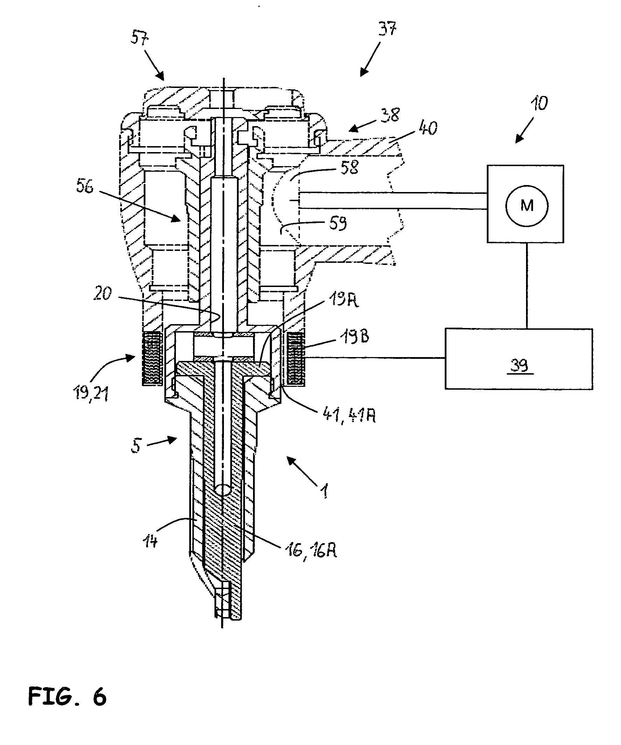

Die Figuren 1, 2A und 2B zeigen einen Bohrer oder ein Bohrwerkzeug 5 wie es für eine Vorrichtung 1, für den Schnellstopp eines medizinischen, insbesondere dentalen, Bohrwerkzeugs 5 verwendbar ist. Das Bohrwerkzeug 5 weist einen länglichen Körper 12 mit einer Längsausdehnung L auf, der sich entlang einer Längsachse oder Mittelachse oder Rotationsachse 13 erstreckt. Die Enden des Körpers 12 sind als Anschlussende 9 zur Verbindung mit einem Antrieb 10 (siehe Figur 6) und einem abrasiven Arbeitsende 11 zum Materialabtrag ausgebildet. Der Körper 12 besteht des Weiteren aus einer hohlen Außenhülse 14 (siehe Figur 2B) und einem darin aufgenommenen Fühler 16, der relativ zur Außenhülse 14 entlang der Längsachse 13 bewegbar ist.

Der Fühler 16 ist als länglicher, sich entlang der Längsachse 13 erstreckender Stift 16A mit einem ersten, dem Anschlussende 9 zugewandten Ende 18A und einem zweiten, dem Arbeitsende 11 zugewandten Ende 18B ausgebildet. Ein Teil des Fühlers 16 ist durch eine Öffnung 17 (siehe Figur 2B) der Außenhülse 14 am Arbeitsende 11 aus der Außenhülse 14 bewegbar, jedoch ist in verbautem Zustand das erste, dem Anschlussende 9 zugewandte Ende 18A des Fühlers 16 immer im Inneren der hohlen Außenhülse 14 des Bohrwerkzeugs 5 aufgenommen.

Im Folgenden wird das abrasive Arbeitsende 11 des Bohrwerkzeugs 5 beschrieben, das auch einen eigenen, separaten erfinderischen Aspekt darstellt. Ein derartiges abrasives Arbeitsende 11 kann somit auch bei anderen Bohrern oder Bohrwerkzeugen verwendet werden, welche insbesondere keine oder andere als die unten noch im Detail zu beschreibenden Sensoren zur Detektion einer Relativbewegung zwischen der Außenhülse und dem Fühler haben.

Am abrasiven Arbeitsende 11 des Bohrwerkzeugs 5 ist ein Steg 43 vorgesehen, der integraler Teil der hohlen Außenhülse 14 oder mit dieser verbunden ist. Der Steg 43 überspannt die Öffnung 17 der Außenhülse 14 oder die an die Öffnung 17 anschließende im Wesentlichen zylindrische Innenbohrung der Außenhülse 14. Alternativ erstreckt sich der Steg 43 von einer Seite der Außenhülse 14 über die Öffnung 17 oder die Innenbohrung der Außenhülse 14 zu einer zweiten Seite der Außenhülse 14. Bevorzugt ist der Steg 43 mittig an der Außenhülse 14 angeordnet, so dass die Längsachse 13 des Bohrwerkzeugs 5 durch den Steg 43 hindurchtritt (siehe insbesondere Figur 2A). Bevorzugt weist der Steg 43 zwei im Wesentlichen flächig oder plan ausgebildete Seitenwände oder Seitenflächen 45A, 45B auf. Jede Seitenfläche 45A, 45B endet an ihrem distalen, dem Anschlussende 9 abgewandten Ende in einer Kante 46A, 46B (siehe Figur 1), an die eine freie Endfläche 47 des Stegs 43 anschließt. Wie insbesondere aus der Figur 2A zu erkennen ist, ist die freie Endfläche 47 im Querschnitt bevorzugt gebogen oder kreisbogenförmig ausgebildet und weist besonders bevorzugt an ihrem Apex einen Einstich oder Rücksprung 48 mit Kanten auf.

An dem Steg 43 sind eine oder mehrere abrasive Elemente vorgesehen, insbesondere Schneiden. Gemäß einem bevorzugten Ausführungsbeispiel ist jede der beiden Kanten 46A, 46B, insbesondere durch den Einstich 48, in zwei Teile 46A1, 46A2, 46B1, 46B2 unterteilt, wobei jeweils nur eine Teilkante 46A1, 46A2 an der Seitenfläche 45A und eine Teilkante 46B1, 46B2 an der Seitenfläche 45B als abrasive Schneide oder Schneidkante ausgebildet ist. Besonders bevorzugt liegen die beiden als Schneidkanten ausgebildeten Teilkanten (bezogen auf die Mittelachse 13) einander diametral gegenüber, so wie dies beispielhaft in der Figur 1 dargestellt ist: Als Schneidkanten sind die Teilkante 46A1 an der Seitenfläche 45A und die Teilkante 46B2 an der gegenüber liegenden Seitenfläche 45B ausgebildet. Insbesondere sind als Schneiden die jeweils an die Schneidkanten 46A1, 46B2 anschließenden Kanten des Einstichs 48 sowie bevorzugt auch zumindest Teile der an die Schneidkanten 46A1, 46B2 anschließenden und die Seitenflächen 45A, 45B lateral begrenzenden Seitenkanten 44A (siehe Figur 1) und 44B (siehe Figur 2B) ausgebildet. Gemäß einem Ausführungsbeispiel ist jeweils ein unmittelbar an die Schneidkanten 46A1, 46B2 angrenzender Abschnitt der Seitenkanten 44A, 44B mit einer Länge von etwa 0,5 mm - 3,0 mm, bevorzugt von etwa 1,0 mm, als Schneide ausgebildet. Die Teilkanten 46A2, 46B1 sind nicht als Schneidkanten ausgebildet und bevorzugt etwas abgerundet und / oder axial (bezogen auf die Längsachse 13) und relativ zu den Schneidkanten 46A1, 46B2 etwas zurück versetzt.

Zur Ausbildung der abrasiven Seitenkanten 44A, 44B sind an dem zylindrischen Außenmantel 49 des Bohrwerkzeugs 5 zwei flächige Rücksprünge 50 (nur einer davon ist in der Figur 1 zu erkennen) vorgesehen, die bei Betrieb des Bohrwerkzeugs 5 auch zur Abfuhr von durch die Schneidkanten abgelösten Materialspänen, insbesondere Knochenspänen, dienen.

Der Steg 43 ist in den Figuren 1, 2A, 2B als gerades, "I"-förmiges Element mit zwei Kanten 46A, 46 B dargestellt. Selbstverständlich kann der Steg jedoch auch andere Formen aufweisen und / oder mehr Kanten aufweisen, um eine höhere Abrasivität zu erreichen. Zum Beispiel kann der Steg 43 "Y"-förmig ausgebildet sein und somit zumindest drei Schneidkanten aufweisen (an jedem der drei Arme des "Y" eine Schneidkante) oder der Steg 43 kann "X"-förmig ausgebildet sein und somit zumindest vier Schneidkanten aufweisen (an jedem der vier Arme des "X" eine Schneidkante). Gemäß den Figuren 1, 2A, 2B sind die beiden Schneidkanten 46A1, 46B2 um die Breite des Stegs 43 versetzt zueinander angeordnet. Alternativ ist es auch möglich den Steg derart auszuführen, dass die beiden Schneidkanten 46A1, 46B2 eine durchgehende Gerade bilden.

Gemäß dem in den Figuren 1, 2A und 2B dargestellten Ausführungsbeispiel dreht sich der Fühler 16 mit der Außenhülse 14 mit, wenn diese in Drehung versetzt wird, wozu eine Drehmitnahmevorrichtung 51 an dem Bohrwerkzeug 5 vorgesehen ist. Gemäß einem bevorzugten Ausführungsbeispiel sind die Seitenflächen 45A, 45B des Stegs 43 Teil der Drehmitnahmevorrichtung 51, welche die vom Antrieb 10 erzeugte und über das Anschlussende 9 auf die hohlen Außenhülse 14 übertragenen Drehbewegung an den in der Außenhülse 14 aufgenommenen Fühler 16 weiterleiten. Der Fühler 16 weist an seinem zweiten Ende 18B eine Fühlerspitze 36 auf, die exzentrisch zur Längsachse 13 angeordnet ist. An der Fühlerspitze 36 ist zumindest eine im Wesentlichen flächige oder plane Kontaktfläche 36A vorgesehen, die kontaktierend an einer der beiden Seitenflächen 45A, 45B des im Wesentlichen zentral angeordneten Stegs 43 anliegt. Über diesen Kontakt zwischen den Flächen 36A und 45A oder 45B wird die Drehbewegung von der Außenhülse 14 auf den Fühler 16 übertragen. Selbstverständlich können auch andere Arten von Drehmitnahmevorrichtungen 51 vorgesehen sein, zum Beispiel in Form eines Polygons, insbesondere eines Hexagons, wobei ein Abschnitt des Fühlers 16 eine polygone Außenform aufweist und ein korrespondierender Abschnitt der Innenwand der hohlen Außenhülse 14 ebenfalls eine polygone Form hat.

Gemäß dem in den Figuren 1, 2A und 2B dargestellten Ausführungsbeispiel weist der Fühler 16 kein abrasives Element für den Materialabtrag auf und löst somit kein Material von dem zu bearbeitenden Gegenstand, insbesondere Gewebe. Demgemäß ist die Fühlerspitze 36 abgerundet. Selbstverständlich wäre es jedoch gemäß einem anderen Ausführungsbeispiel auch möglich, den Fühler mit einem abrasiven Element für den Materialabtrag zu versehen, insbesondere mit einer Schneidspitze am distalen, dem Material 55 zugewandten Ende.

Um den Fühler 16 und gegebenenfalls weitere Bauteil im Inneren der hohlen Außenhülse 14 des Bohrwerkzeugs 5 anordnen zu können, ist die Außenhülse 14 zweiteilig aufgebaut, wobei die beiden Teile 14A, 14B über geeignete Verbindungsvorrichtungen miteinander verbindbar sind, insbesondere lösbar verbindbar sind. Die Verbindungsvorrichtungen sind zum Beispiel als Schraub-, Steck- oder Bajonettverbindungen ausgebildet. Die beiden Außenhülsenteile 14A, 14B können aus unterschiedlichen oder gleichen Materialien hergestellt sein, zum Beispiel aus Metall, insbesondere Stahl, und / oder Kunststoff, wobei insbesondere jener Außenhülsenteil 14B mit dem abrasiven Ende 11 bevorzugt aus Metall hergestellt ist und gegebenenfalls jener Außenhülsenteil 14A, der den im Weiteren noch zu beschreibenden elektromagnetischen Sensor 19 umgibt, zumindest teilweise aus Kunststoff besteht, um die Funktion des Sensors 19 nicht oder weniger zu beeinträchtigen. Insbesondere wenn der elektromagnetische Sensor 19 als induktiver Sensor 21 ausgebildet ist, ist es von Vorteil zumindest jenen Teil der Außenhülse 14, der im Bereich des induktiven Sensors 21 vorgesehen ist, aus einem nicht-magnetischen Material herzustellen, zum Beispiel aus Aluminium, aus nichtmagnetischem Stahl, aus Kunststoff oder aus Keramik, zum Beispiel aus keramischen Material mit Zirkonium.

In den Figuren 2A, 2B ist eine Vorrichtung 1 für den Schnellstopp eines medizinischen, insbesondere dentalen, Bohrwerkzeugs, insbesondere des in der Figur 1 dargestellten Bohrwerkzeugs 5, dargestellt. Die Vorrichtung 1 umfasst neben dem Bohrwerkzeug 5 auch noch einen elektromagnetischen Sensor 19, der zur Detektion einer Relativbewegung zwischen der Außenhülse 14 und dem Fühler 16 ausgebildet ist. Der Sensor 19 ist entlang oder innerhalb der Längsausdehnung L des Körpers 12 des Bohrwerkzeugs 5 angeordnet. Der Sensor 19 überragt somit die beiden Enden 9, 11 des Bohrwerkzeugs 5 nicht oder nicht wesentlich. Es ist des Weiteren zu erkennen, dass ein Teil des Sensors 19 in dem Bohrwerkzeug 5 und ein weiterer Teil des Sensors 19 außerhalb des Bohrwerkzeugs 5 angeordnet ist.

Gemäß den Figuren 2A, 2B ist der elektromagnetische Sensor 19 als berührungsloser, induktiver Sensor 21 ausgebildet, der zumindest eine Spule 22, die außerhalb des Bohrwerkzeugs 5, jedoch nahe dessen Außenmantel 49 angeordnet ist, und einen Spulenkern 23 umfasst. Die zumindest eine Spule 22 kann das Bohrwerkzeug 5 zum Beispiel ringförmig umgeben. Der Spulenkern 23 umfasst ein Magnetelement, das relativ zur Spule 22 bewegbar ist. Das Magnetelement ist entweder ein separates Element, das mit dem Fühler 16 fest verbunden ist und gemeinsam mit dem Fühler 16 bewegbar ist, oder das Magnetelement wird durch den Fühler 16 gebildet, zum Beispiel in dem der Fühler 16 aus einem magnetischen Material hergestellt ist oder bei der Herstellung magnetisiert wurde, so wie dies für die Figuren 2A und 2B zutrifft. Der Spulenkern 23 ist insbesondere durch das erste Ende 18A des Fühlers 16, das im Inneren der hohlen Außenhülse 14 aufgenommen ist, gebildet.

Der Fühler 16 bzw. der Spulenkern 23 sind durch ein Federelement 15 in Richtung des abrasiven Endes 11 des Bohrwerkzeugs 5 vorgespannt, so dass zumindest ein Teil des Fühlers 16 oder der Fühlerspitze 36 durch die Öffnung 17 nach außen ragt oder die hohle Außenhülse 14, insbesondere den Steg 43, überragt. Das Federelement 15 ist in den Figuren 2A, 2B als aus einem elastischen Material, insbesondere aus elastischem Kunststoff, bestehender Schlauchabschnitt dargestellt, es kann jedoch selbstverständlich auch andere Federn umfassen, insbesondere Spiral- oder Blattfedern, zum Beispiel auch aus Metall.

Die Detektion einer Relativbewegung zwischen der Außenhülse 14 und dem Fühler 16 durch den induktiven Sensor 21 läuft wie folgt ab: Wirkt auf den Fühler 16 eine Kraft, die der Federkraft des Federelements 15 entgegengesetzt ist und die groß genug ist, das Federelement 15 zu komprimieren, dann bewegen sich der Fühler 16 bzw. der Spulenkern 23 bzw. das Magnetelement axial entlang der Längsachse 13 in Richtung des Anschlussendes 9 des Bohrwerkzeugs 5. Der Spulenkern 23 dringt damit in die Spule 22 ein oder dringt weiter in die Spule 22 ein. Die Spule 22 ist mit einer Wechselspannungsquelle verbunden und wird von dieser mit einer Wechselspannung versorgt. Mit dem (weiteren) Eindringen des Spulenkerns 23 oder des Magnetelements in die Spule 22 ändert sich somit deren Induktivität, woraus eine Relativbewegung zwischen der Außenhülse 14 des Bohrwerkzeugs 5 und dem Spulenkern 23 oder dem Fühler 16 abgeleitet werden kann. Die Induktivitätsänderung wird in Form eines Sensorsignals an eine mit dem Sensor 19 verbundene Steuer- und / oder Regelvorrichtung 39 (siehe Figur 6) geleitet, welche auf Basis des Sensorsignals den Antrieb 10 für das Bohrwerkzeug 5 stoppt.

Eine Relativbewegung zwischen der Außenhülse 14 und dem Fühler 16 tritt insbesondere dann auf, wenn das Bohrwerkzeug 5 an einen Übergangsbereich zwischen zwei Materialien mit unterschiedlichen Härten gelangt, insbesondere im medizinischen Bereich an einen Übergangsbereich von einem harten Gewebe, zum Beispiel Knochen, und einem weichen Gewebe, zum Beispiel eine Membran, Muskel- oder Bindegewebe, Gehirnmasse oder gar in einen (gegebenenfalls mit einem Flüssigkeit oder einem Gas gefüllten) Hohlraum. Entsprechende Anwendungsbeispiele, auch für die dentale Anwendung, insbesondere beim Durchbohren des Oberkieferknochens im Rahmen einer Sinusbodenelevation, sind in der eingangs genannten Patentanmeldung DE 10 2008 032 704 A1 beschrieben, so dass diesbezüglich auf diese Schrift verwiesen wird.

Das erste Ende 18A des Fühlers 16 ist bevorzugt radial (bezogen auf die Längsachse 13) verbreitert oder als Flansch oder flanschartige Verbreiterung ausgebildet, um damit eine stabile und ausreichend große Kontaktfläche für das Federelement 15 zu schaffen und / oder eine verbesserte Funktion des Sensors 19 zu bewirken, in dem die innerhalb und außerhalb des Bohrwerkzeugs 5 angeordneten Teile des Sensors 19 räumlich möglichst nahe zueinander zu positionieren. Zumindest das radial verbreiterte Ende 18A, bevorzugt auch das Federelement 15, sind in einem ebenfalls radial verbreiterten Abschnitt des Körpers 12 oder der Außenhülse 14 des Bohrwerkzeugs 5 aufgenommen.

Das Bohrwerkzeug 5 weist einen sich entlang der Längsachse 13 erstreckenden Leitungskanal 20 für ein Behandlungsfluid und / oder Kühlfluid auf. Der Leitungskanal 20 beginnt an einer Öffnung 52 am äußersten Ende des Anschlussendes 9. Über diese Öffnung 52 ist der Leitungskanal 20 mit einer Fluidquelle verbindbar, die zum Beispiel eine physiologische Kochsalzlösung oder ein Anästhetikum oder eine Flüssigkeit zum Anheben einer Membran zur Verfügung stellt. Die Verbindung zur Fluidquelle wird zum Beispiel über ein Röhrchen bewerkstelligt, das durch die Öffnung 52 in den Leitungskanal 20 einführbar ist und das, gegebenenfalls über eine weitere Leitung, mit der Fluidquelle verbunden ist. Gegebenenfalls sind an dem Röhrchen, oder an dem Bohrwerkzeug 5, zum Beispiel an der Öffnung 52 oder im Leitungskanal 20, ein oder mehrere Dichtelemente, zum Beispiel O-Ringe, vorgesehen, die ein Austreten des Fluids aus dem Röhrchen oder dem Leitungskanal 20 verhindern.

Der Leitungskanal 20 verläuft entlang der Längsachse 13 im Inneren der Außenhülse 14 und wird im an die Öffnung 52 anschließenden Abschnitt durch die Innenbohrung 31 der hohlen Außenhülse 14 gebildet. Daran anschließend durchtritt der Leitungskanal 20 das Federelement 15 oder setzt sich in diesem fort oder führt daran vorbei. In dem Ausführungsbeispiel gemäß den Figuren 2A, 2B weist das Federelement 15 eine Durchbohrung 53 auf, so dass das Fluid durch das Federelement 15 und anschließend weiter in eine Bohrung 32 des Fühlers 16 fließen kann. Die Bohrung 32 erstreckt sich ebenfalls axial durch den Fühler 16 entlang der Längsachse 13 und mündet an ihrem der Fühlerspitze 36 zugewandten Ende in eine Querbohrung 35. Die Querbohrung 35 durchsetzt den Fühler 16 radial zur Längsachse 13 und weist zumindest eine Öffnung 54 auf, die in einen Spalt 34 mündet, insbesondere in einen Ringspalt, der durch eine Beabstandung der Außenwand des Fühlers 16 von der Innenwand der hohlen Außenhülse 14 entsteht. Der Spalt 34 endet schließlich in der Öffnung 17, durch die das Fluid aus dem Bohrwerkzeug 5, insbesondere aus der Außenhülse 14, austritt. Alternativ oder zusätzlich ist es möglich, die Bohrung 32 bis zum zweiten Ende 18B des Fühlers 16 zu führen, so dass zumindest ein Teil des Fluids über eine Öffnung im Fühler 16 abgegeben wird.

Um den Spalt 34 bilden zu können, weist der Fühler 16 zwei axial hintereinander angeordnete Abschnitte auf: Einen Führungsabschnitt 33A, dessen Durchmesser in etwa der lichten Weite der hohlen Außenhülse 14 entspricht, so dass der Führungsabschnitt 33A an der Innenwand der hohlen Außenhülse 14 gelagert ist, und einen zweiten Abschnitt 33B, dessen Durchmesser geringer ist als die lichte Weite der hohlen Außenhülse 14, so dass der Fühler 16 in diesem Abschnitt 33B von der Innenwand der Außenhülse 14 beabstandet ist, wodurch der Spalt 34 entsteht. Der Führungsabschnitt 33A dient zum Lagern des Fühlers 16 an der Innenwand der hohlen Außenhülse 14 und zum Führen oder Gleiten des Fühlers 16 in der Außenhülse 14.

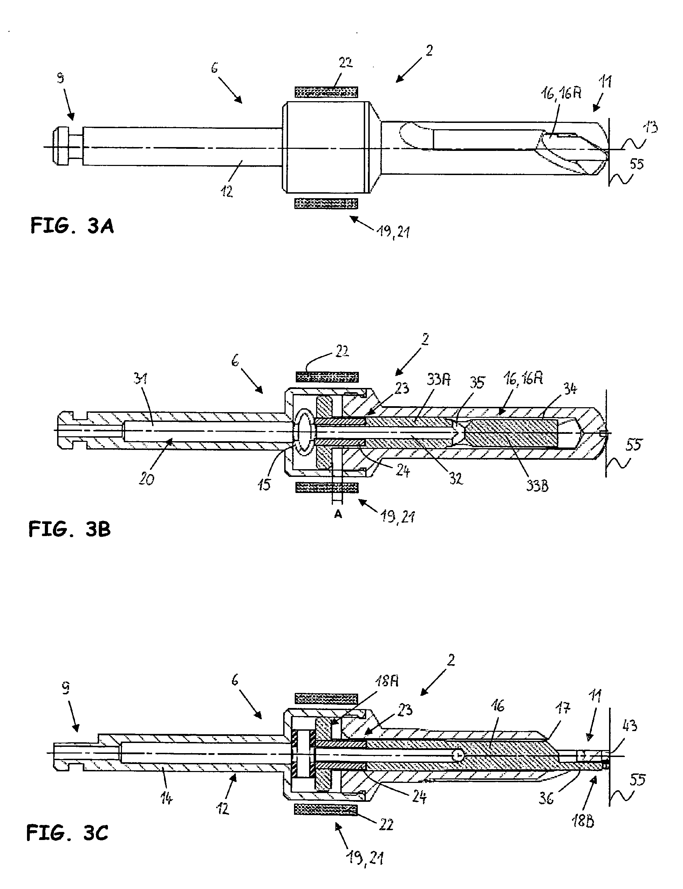

Die Figuren 3A - 3C, 4 und 5 zeigen weitere Vorrichtungen 2, 3, 4 für den Schnellstopp eines medizinischen, insbesondere dentalen, Bohrwerkzeugs 6, 7, 8. Sowohl die Vorrichtungen 2, 3, 4 als auch die Bohrwerkzeuge 6, 7, 8 ähneln in ihrem Aufbau und in ihren Funktionen der im Vorstehenden beschriebenen Schnellstopp-Vorrichtung 1 und dem Bohrwerkzeug 5, so dass gleiche Bauteile mit den gleichen Bezugszeichen versehen sind. Im Folgenden werden daher bevorzugt die abweichenden Merkmale der Schnellstopp-Vorrichtungen 2, 3, 4 beschrieben.

Die in den Figuren 3A - 3C dargestellte Schnellstopp-Vorrichtung 2 umfasst einen elektromagnetischen Sensor 19 zur Detektion einer Relativbewegung zwischen der Außenhülse 14 und dem Fühler 16. Der Sensor 19 ist wiederum als induktiver Sensor 21 ausgebildet, wobei der Spulenkern 23 einen weichmagnetischen Ferritkörper 24 umfasst, welcher bei einer Relativbewegung zwischen dem Fühler 16 und der hohlen Außenhülse 14 bzw. zwischen der Spule 22 und dem Spulenkern 23 die Induktivität der zumindest einen Spule 22 ändert. Der Ferritkörper 24 ist mit dem Fühler 16 verbunden, so dass er sich gemeinsam mit dem Fühler 16 bewegt. Bevorzugt ist der Ferritkörper 24 in einer Ausnehmung des Fühlers 16 aufgenommen. Bevorzugt ist der Ferritkörper 24 als Zylinder oder Hohlzylinder ausgebildet, der konzentrisch am Fühler 16 angeordnet ist. Besonders bevorzugt verläuft der Leitungskanal 20 durch den hohlzylindrischen Ferritkörper 24. Selbstverständlich kann der Ferritkörper jedoch auch andere Formen aufweisen, zum Beispiel als Ferritstab am Fühler 16 befestigt sein.

In den Figuren 3A - 3C ist des Weiteren zu erkennen, dass das Bohrwerkzeug 6 auf ein hartes Material 55 aufgesetzt ist, zum Beispiel ein Knochengewebe. Diese Situation liegt zum Beispiel vor, wenn der Anwender das Bohrwerkzeug 6 vor Beginn des Bohrens auf das Material 55 aufsetzt oder während des Bohrens in dem Material 55. Der Fühler 16 und somit der Spulenkern 23 sind entgegen der Federkraft des Federelements 15 in die hohle Außenhülse 14 (in Richtung des Anschlussendes 9) gedrängt, so dass das freie Ende der Fühlerspitze 36 im Wesentlichen mit dem Steg 43 fluchtet (siehe insbesondere Figur 3C). Das Federelement 15 ist demgemäß komprimiert. Wird das Bohrwerkzeug 6 von dem Material 55 abgehoben oder durchbricht das Bohrwerkzeug 6 das Material 55 oder steht das Bohrwerkzeug 6 kurz davor, das Material 55 zu durchbrechen, dann entspannt sich das Federelement 15 und bewegt den Fühler 16 und den Spulenkern 23 in Richtung des abrasiven Endes 11, so dass zumindest ein Teil der Fühlerspitze 36 die hohle Außenhülse 14, insbesondere den Steg 43, überragt (so wie dies in den Figuren 2A, 2B dargestellt ist). Das Gleiten des Fühlers 16 relativ zur Außenhülse 14 bewirkt die Induktivitätsänderung der Spule 22 und führt zum unverzüglichen Stoppen des Antriebs des Bohrwerkzeugs 5, 6, so wie dies im Vorherstehenden schon beschrieben wurde. Um ein eindeutiges und gut verwertbares Sensorsignal zu erhalten, sollte die Wegänderung A (siehe Figur 3B) des Fühlers 16 oder Spulenkerns 23 zumindest 0,2 mm, bevorzugt zumindest 0,3 mm, betragen.

Gemäß einem Ausführungsbeispiel beträgt der Durchmesser der Spule 22 des induktiven Sensors 21 etwa 5 ― 8 mm, die Windungsanzahl der Spule 22 etwa 20 - 40, die Länge des Ferritkörpers 24 etwa 2 ― 3 mm und die angelegte Effektivspannung in etwa 0,7 - 1,0 V.

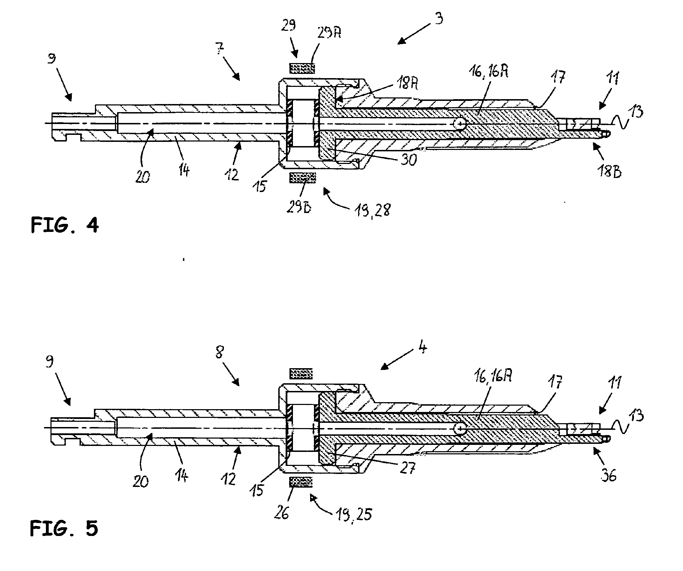

Die in der Figur 4 dargestellte Vorrichtung 3 für den Schnellstopp eines medizinischen, insbesondere dentalen, Bohrwerkzeugs 7 weist einen berührungslosen, elektromagnetischen Sensor 19 zur Detektion einer Relativbewegung zwischen der Außenhülse 14 und dem Fühler 16 auf, der als kapazitiver Sensor 28 ausgebildet ist. Der kapazitive Sensor 28 umfasst zumindest zwei metallischen Elektroden 29, 30, die einen Kondensator bilden, wobei eine Elektrode 30 durch die Relativbewegung des Fühlers 16 zur Außenhülse 14 relativ zu einer anderen Elektrode 29 bewegbar ist. Die Elektroden 29, 30 sind über elektrische Leitungen mit einer Wechselspannungsquelle verbunden und bilden einen hochfrequenten Schwingkreis, an dem ein elektrisches Feld erzeugt wird. Durch die Relativbewegung der beiden Elektroden 29, 30 kommt es zu einer Kapazitätsänderung und damit im Schwingkreis zu einer Veränderung der Verstärkung. Die Kapazitätsänderung bzw. die Veränderung der Verstärkung wird in Form eines Sensorsignals an eine mit dem Sensor 28 verbundene Steuer- und / oder Regelvorrichtung 39 (siehe Figur 6) geleitet, welche auf Basis des Sensorsignals den Antrieb 10 für das Bohrwerkzeug 7 stoppt.

Bevorzugt ist eine Elektrode 30 des Kondensators mit dem Fühler 16 verbunden oder durch den Fühler 16, insbesondere durch einen metallischen Abschnitt des Fühlers 16, gebildet. Die Elektrode 30 ist somit im Bohrwerkzeug 7 aufgenommen. Besonders bevorzugt ist die Elektrode 30 an dem radial verbreiterten Ende 18A des Fühlers 16 vorgesehen oder wird durch dieses Ende 18A gebildet. Eine andere Elektrode 29 ist außerhalb des Bohrwerkzeugs 7, entlang oder innerhalb der Längsausdehnung des Körpers 12 des Bohrwerkzeugs 7 angeordnet. Der kapazitive Sensor 28 überragt somit die beiden Enden 9, 11 des Bohrwerkzeugs 7 nicht oder nicht wesentlich.

Gemäß einem besonders bevorzugten Ausführungsbeispiel weist der kapazitive Sensor 28 zumindest zwei im Wesentlichen plattenförmige Elektroden 29A, 29B sowie eine mit dem Fühler 16 entlang der Längsachse 13 des Bohrwerkzeugs 7 und relativ zu den zwei im Wesentlichen plattenförmigen Elektroden 29A, 29B bewegbare Messelektrode 30 auf. Die plattenförmigen Elektroden 29A, 29B sind außerhalb des Bohrwerkzeugs 7, entlang oder innerhalb der Längsausdehnung des Körpers 12 des Bohrwerkzeugs 7 angeordnet. Die beiden plattenförmige Elektroden 29A, 29B sind insbesondere als Brückenschaltung ausgebildet.

Die in der Figur 5 gezeigte Vorrichtung 4 für den Schnellstopp eines medizinischen, insbesondere dentalen, Bohrwerkzeugs 8 weist einen elektromagnetischen Sensor 19 zur Detektion einer Relativbewegung zwischen der Außenhülse 14 und dem Fühler 16 auf, der als Magnetsensor 25 ausgebildet ist. Der Fühler 16 weist ein Magnetelement 27 auf, das, wie für die Figuren 2A, 2B beschrieben, entweder fest mit dem Fühler 16 verbunden ist, um sich gemeinsam mit dem Fühler 16 zu bewegen, oder das durch den Fühler 16 gebildet ist, zum Beispiel in dem der Fühler 16 aus einem magnetischen Material hergestellt ist oder bei der Herstellung magnetisiert wurde, so wie dies für die Figur 5 zutrifft. Das Magnetelement 27 ist somit wiederum im Inneren der hohlen Außenhülse 14 aufgenommen.

Außerhalb des Bohrwerkzeugs 8, jedoch entlang oder innerhalb der Längsausdehnung des Körpers 12 des Bohrwerkzeugs 7 ist zumindest ein Magnetsensor 26 angeordnet, zum Beispiel ein Hall-Sensor oder ein Reed-Sensor, der einen magnetischen Parameter des Magnetelements 27 detektiert, zum Beispiel die Magnetfeldstärke. Durch die Relativbewegung zwischen der Außenhülse 14 und dem Fühler 16 kommt es zu einer Abstandsänderung zwischen dem Magnetelement 27 und dem Magnetsensor 26 und somit zu einer Änderung des Wertes des magnetischen Parameters an dem Magnetsensor 26. Zum Beispiel nimmt die Magnetfeldstärke des vom Magnetelement 27 erzeugten Magnetfelds am Magnetsensor 26 ab, wenn der Fühler 16 mit dem Magnetelement 27 beim Durchbrechen des gebohrten Materials in Richtung des abrasiven Endes 11 gleitet. Die Veränderung des Wertes des magnetischen Parameters wird wiederum in Form eines Sensorsignals an eine mit dem Sensor 25 verbundene Steuer- und / oder Regelvorrichtung 39 (siehe Figur 6) geleitet, welche auf Basis des Sensorsignals den Antrieb 10 für das Bohrwerkzeug 8 stoppt.

Um die Funktion des Sensors 25 zu unterstützen, ist bevorzugt jener Teil der hohlen Außenhülse 14, an dem der Sensor 25 angeordnet ist, aus nicht metallischem Material hergestellt, zum Beispiel aus Kunststoff.

In der Figur 6 ist eine medizinische, insbesondere dentale, Behandlungsvorrichtung 37 dargestellt, die beispielhaft die Vorrichtung 1 für den Schnellstopp eines medizinischen, insbesondere dentalen, Bohrwerkzeugs 5 umfasst. Selbstverständlich kann die Behandlungsvorrichtung 37 auch die anderen, im Vorstehenden beschriebenen Schnellstopp-Vorrichtungen 2, 3, 4 und die entsprechenden Bohrwerkzeuge 6, 7, 8 aufweisen, so dass die folgende Beschreibung der Figur 6 rein exemplarisch ist und in entsprechender Weise auf die anderen Schnellstopp-Vorrichtungen 2, 3, 4 und die anderen Bohrwerkzeuge 6, 7, 8 zutrifft.

Die Behandlungsvorrichtung 37 umfasst des Weiteren einen Antrieb 10 für das Bohrwerkzeug 5, ein Handstück 38 zum Anschluss des Bohrwerkzeugs 5 und eine Steuer-und / oder Regelvorrichtung 39 zum Empfang eines von dem elektromagnetischen Sensor 19 erzeugten Sensorsignals und zum Stoppen des Antriebs 10. Bevorzugt kann die Behandlungsvorrichtung 37 Vorrichtungen zum Verarbeiten des Sensorsignals des Sensors 19 aufweisen, zum Beispiel zum Glätten, Filtern oder Verstärken des Signals, wobei diese Vorrichtungen insbesondere in der Steuer- und / oder Regelvorrichtung 39 vorgesehen sind oder als Teil der Steuer- und / oder Regelvorrichtung 39 ausgebildet sind.

Das Handstück 38 ist bevorzugt als gewinkeltes Handstück oder Winkelstück mit einer seitlich angeordneten Werkzeug-Einstecköffnung 41 ausgebildet. Das Handstück 38 weist eine hohle Außen- oder Griffhülse 40 auf, in deren Kopfabschnitt eine in eine Arbeitsbewegung versetzbare Werkzeuganschlussvorrichtung oder Spannzange 56 vorgesehen ist. Die Werkzeuganschlussvorrichtung 56 ist insbesondere in Drehung versetzbar und bevorzugt in Wälzlagern gelagert. Mittels einer Lösevorrichtung 57 kann das Bohrwerkzeug 5 wieder aus der Werkzeuganschlussvorrichtung 56 gelöst werden. Insbesondere ist die Werkzeuganschlussvorrichtung 56 als formschlüssige Werkzeuganschlussvorrichtung ausgebildet, zum Beispiel als Aufnahme für einen 2,35 mm Standardbohrer.

Die Werkzeuganschlussvorrichtung 56 ist operativ mit dem Antrieb 10 verbunden, so dass der Antrieb 10 die Werkzeuganschlussvorrichtung 56 und das Bohrwerkzeug 5 in eine Arbeitsbewegung, insbesondere in Rotation, versetzt. Der Antrieb 10 umfasst zum Beispiel einen ansteuerbaren Motor, insbesondere einen Elektromotor, eine oder mehrere Wellen, ein Getriebe, Kupplungen und / oder Zahnräder. Die unmittelbare Übertragung der Antriebsbewegung vom Antrieb 10 auf die Werkzeuganschlussvorrichtung 56 erfolgt über ein am Antrieb vorgesehenes Zahnrad 58 und ein an der Werkzeuganschlussvorrichtung 56 befestigtes Ritzel 59, das mit dem Zahnrad 58 kämmt.

Die Steuer- und / oder Regelvorrichtung 39 ist über elektrische Leitungen und / oder Signalleitungen mit dem Antrieb 10, insbesondere mit dem Motor, und mit dem Sensor 19 verbunden. Die Leitung für den Sensor 19 kann dabei innerhalb des Handstücks 38, insbesondere in der Außenhülse 40, und / oder außerhalb des Handstücks 38 verlaufen.

Die Steuer- und / oder Regelvorrichtung 39 weist eine Energiequelle auf oder ist mit einer Energiequelle verbunden und versorgt den Sensor 19 mit elektrischer Energie, insbesondere mit der benötigten Wechselspannung. Die Steuer- und / oder Regelvorrichtung 39 steuert und / oder regelt den Betrieb des Motors des Antriebs 10, insbesondere versorgt sie den Motor mit Energie oder bestimmt die Drehzahl und / oder das Drehmoment und stoppt den Motor, wenn der elektromagnetische Sensor 19 eine Relativbewegung zwischen der Außenhülse 14 und dem Fühler 16 detektiert. Bevorzugt umfasst die Steuer- und / oder Regelvorrichtung 39 einen Mikrocontroller oder Microcomputer. An der Steuer- und / oder Regelvorrichtung 39 können des Weiteren Stellelemente zum Auswählen oder Einstellen von Betriebsparametern der Behandlungsvorrichtung 37 durch den Anwender und / oder eine Anzeige zum Anzeigen von Betriebsparametern vorhanden sein.

Da während des Bohrvorgangs geringe Relativbewegung zwischen der Außenhülse 14 und dem Fühler 16 auftreten können, welche bereits ein Sensorsignal generieren, ohne dass das Bohrwerkzeug das Material 55 bereits durchbricht oder kurz davor steht, das Material 55 zu durchbrechen, ist bevorzugt in der Steuer- und / oder Regelvorrichtung 39 ein Schwellenwert festgelegt oder durch den Anwender festlegbar, den das Sensorsignal des Sensors 19 überschreiten muss, damit die Steuer- und / oder Regelvorrichtung 39 den Antrieb 10 stoppt.

Gemäß einem Ausführungsbeispiel ist der Sensor 19 im Bereich der Werkzeug-Einstecköffnung 41 des Handstücks 38 vorgesehen.

Gemäß dem Ausführungsbeispiel der Figur 6 ist zumindest ein Teil 19B des elektromagnetischen Sensors 19, 21, insbesondere direkt, am oder im Handstück 38 angeordnet, insbesondere direkt an oder in der Außenhülse 40 des Handstücks 38 befestigt. Der Teil 19B des Sensors 19 bildet somit einen integralen Teil des Handstücks 38, der mit dem Handstück 38 fest verbundenen ist. Im Gegensatz dazu ist der andere Teil 19A des Sensors 19 fest mit dem Bohrwerkzeug 5 verbunden und bildet einen integralen Teil des Bohrwerkzeugs 5. Wird das Bohrwerkzeug 5 mit dem Handstück 38 verbunden, dann sind die beiden Teile 19A, 19B des Sensors 19 derart angeordnet, dass sie operativ miteinander verbunden sind, um eine Relativbewegung zwischen der Außenhülse 14 und dem Fühler 16 zu detektieren.

Gemäß einem bevorzugten Ausführungsbeispiel weist die Werkzeug-Einstecköffnung 41 einen Abschnitt oder Bereich auf, an dem der mit dem Handstücks 38 integrale Teil 19B des Sensors 19 angeordnet ist, zum Beispiel die Spule 22 des induktiven Sensors 21 oder eine Elektrode 29 (Platte 29A, 29B) des kapazitiven Sensors 28. Besonders bevorzugt weist Werkzeug-Einstecköffnung 41 einen verbreiterten Abschnitt 41A auf, an dem der mit dem Handstücks 38 integrale Teil 19B des Sensors 19 angeordnet ist und der zur Aufnahme des anderen Teils 19A des Sensors 19 dient. Insbesondere ist der verbreiterte Abschnitt 41A derart bemessen, dass der Teil 19A des Sensors 19 oder das erst Ende 18A des Fühlers 16 darin aufnehmbar sind. Alternativ weist das Handstück 38, insbesondere die Außenhülse 40, im Bereich der Werkzeug-Einstecköffnung 41 zumindest einen Fortsatz auf, an dem der mit dem Handstücks 38 integrale Teil 19B des Sensors 19 angeordnet ist.

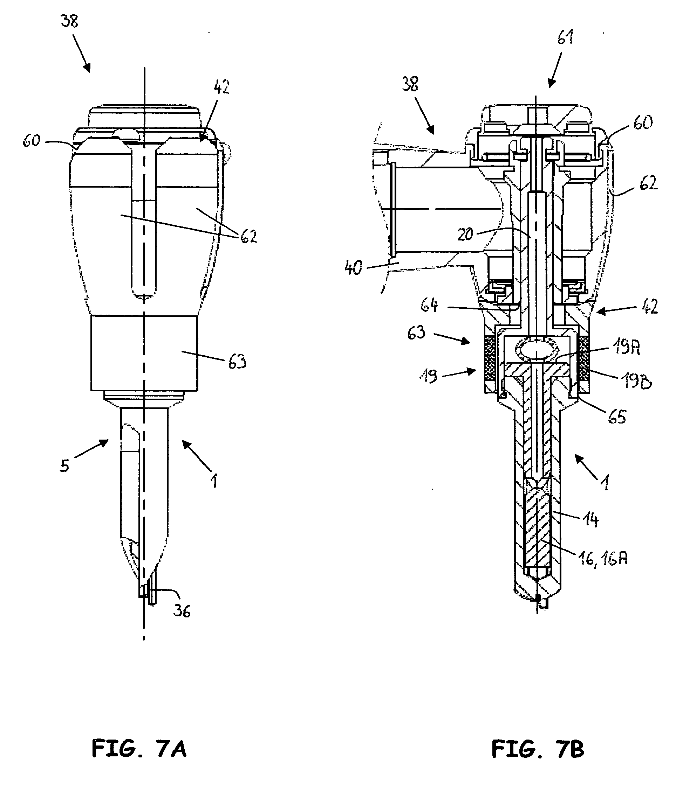

Die Figuren 7A, 7B zeigen ein alternatives Ausführungsbeispiel einer medizinischen, insbesondere dentalen, Behandlungsvorrichtung, bei welcher der gesamte Sensor 19 lösbar mit dem Handstück 38 verbunden ist. Der Sensor 19 ist beispielhaft wieder als induktiver Sensor 21 mit dem Bohrwerkzeug 5 dargestellt, selbstverständlich kann die Behandlungsvorrichtung jedoch auch die anderen, im Vorstehenden beschriebenen Schnellstopp-Vorrichtungen 2, 3, 4 und die entsprechenden Bohrwerkzeuge 6, 7, 8 aufweisen, so dass die folgende Beschreibung der Figuren 7A, 7B wiederum rein exemplarisch ist und in entsprechender Weise auf die anderen Schnellstopp-Vorrichtungen 2, 3, 4 und die anderen Bohrwerkzeuge 6, 7, 8 anwendbar ist. Des Weiteren sind in den Figuren 7A, 7B nur das Handstück 38 und die Schnellstopp-Vorrichtung 1 zu erkennen, jedoch weist auch diese Behandlungsvorrichtung wiederum einen Antrieb, eine Steuer- und / oder Regelvorrichtung und entsprechende elektrische Verbindungen zwischen den Bauteilen auf, wie sie für die Figur 6 beschrieben sind.

Der Sensor 19 weist wiederum zwei Teile 19A, 19B auf, die, wenn sie am Handstück 38 befestigt sind, operativ miteinander verbunden sind, um eine Relativbewegung zwischen der Außenhülse 14 und dem Fühler 16 zu detektieren. Der Teil 19A des Sensors 19 ist fest mit dem Bohrwerkzeug 5 verbunden und bildet einen integralen Teil des Bohrwerkzeugs 5. Durch den Anschluss des Bohrwerkzeugs 5 an das Handstück 38 ist der Teil 19A lösbar mit dem Handstück 38 verbindbar. Der zweite Teil 19B des Sensors 19 ist über eine lösbare Verbindungsvorrichtung 42 direkt am Handstück 38 befestigbar, insbesondere an der Außenhülse 40 des Handstücks 38. Die lösbare Verbindungsvorrichtung 42 kann zum Beispiel als Schraub-, Steck- oder Klemmverbindung ausgebildet sein. Demgemäß sind der erste Teil 19A und der zweite Teil 19B des Sensors 19 an der Außenseite der Außenhülse 40 oder außerhalb der Außenhülse 40 des Handstücks 38 anordenbar.

Gemäß dem in den Figuren 7A, 7B dargestellten bevorzugten Ausführungsbeispiel ist die lösbare Verbindungsvorrichtung 42 als Steckverbindung ausgebildet, die eine Aufnahme 60 zum Einstecken oder Aufnehmen des Kopfabschnitts 61 des Handstücks 38 aufweist. Die Aufnahme 60 umfasst zum Beispiel zwei oder mehrere Federarme 62 zum Befestigen am Kopfabschnitt 61 und einen mit den Federarmen 62 verbundenen Detektionsabschnitt 63 mit dem Teil 19B des Sensors 19. Die Federarme 62 sind insbesondere nach innen, in Richtung der Aufnahme 60 vorgespannt, so dass der Durchmesser der Aufnahme 60 etwas geringer ist als der Durchmesser des Kopfabschnitts 61. Wird der Kopfabschnitt 61 in die Aufnahme 60 gesteckt, so werden die Federarme 62 etwas radial nach außen bewegt und klemmen den Kopfabschnitt 61. Wird der Kopfabschnitt 61 aus der Aufnahme 60 entfernt, so bewegen sich die Federarme 62 wieder radial nach innen in ihre Ausgangsposition.

Der Detektionsabschnitt 63 ist bevorzugt als ringförmiges, hülsenförmiges oder hohlzylindrisches Element ausgebildet, in oder an dem der Teil 19B des Sensors 19 vorgesehen ist, insbesondere die Spule 22 des induktiven Sensors 21 oder eine Elektrode 29 (Platte 29A, 29B) des kapazitiven Sensors 28. Die lösbare Verbindungsvorrichtung 42 ist bevorzugt derart ausgebildet, dass der Detektionsabschnitt 63, insbesondere der Teil 19B des Sensors 19, an der Werkzeug-Einstecköffnung 64 des Handstücks 38 anordenbar ist oder direkt oder bündig daran anschließt. Die lichte Weite der Bohrung 65 des Detektionsabschnitts 63 ist derart bemessen, dass das Bohrwerkzeugs 5 darin aufnehmbar ist, insbesondere der am Bohrwerkzeug 5 vorgesehene Teil 19A des Sensors 19 oder das erst Ende 18A des Fühlers 16.

Bei einem bevorzugten Verfahren zum Bohren eines Materials 55 wird eine im Vorstehenden beschriebene Vorrichtung 1, 2, 3, 4 für den Schnellstopp eines medizinischen, insbesondere dentalen, Bohrwerkzeugs 5, 6, 7, 8 oder eine im Vorstehenden beschriebene medizinische, insbesondere dentale, Behandlungsvorrichtung 37 verwendet, wobei der Antrieb des Bohrwerkzeugs 5, 6, 7, 8 gestoppt wird, wenn der elektromagnetischen Sensor 19 eine, insbesondere einen Schwellenwert übersteigende, Relativbewegung zwischen der Außenhülse 14 und dem Fühler 16 des Bohrwerkzeugs 5, 6, 7, 8 detektiert. Bevorzugt ist das Material 55 ein menschliches oder tierisches Gewebe, bevorzugt ein Knochengewebe, insbesondere ein Kieferknochen.

Eine bevorzugte Verwendung der im Vorstehenden beschriebenen Vorrichtung 1, 2, 3, 4 für den Schnellstopp eines medizinischen, insbesondere dentalen, Bohrwerkzeugs 5, 6, 7, 8 oder einer im Vorstehenden beschriebenen medizinischen, insbesondere dentalen, Behandlungsvorrichtung 37 erfolgt beim Durchbohren eines menschlichen oder tierischen Oberkieferknochens, insbesondere im Rahmen einer Sinusbodenelevation.

## Patentansprüche

1. Vorrichtung (1, 2, 3, 4) für den Schnellstopp eines medizinischen, insbesondere dentalen, Bohrwerkzeugs (5, 6, 7, 8), umfassend:
- Ein Bohrwerkzeug (5, 6, 7, 8) mit einem Anschlussende (9) zur Verbindung mit einem Antrieb (10), einem abrasiven Arbeitsende (11) zum Materialabtrag und einem sich zwischen dem Anschlussende (9) und dem Arbeitsende (11) erstreckenden Körper (12) mit einer sich entlang einer Längsachse (13) des Bohrwerkzeugs (5, 6, 7, 8) erstreckenden Längsausdehnung (L), wobei das Bohrwerkzeug (5, 6, 7, 8) eine hohle Außenhülse (14) aufweist, in welcher ein durch ein Federelement (15) vorgespannter Fühler (16) aufgenommen ist, der relativ zur Außenhülse (14) entlang der Längsachse (13) bewegbar ist, so dass zumindest ein Teil des Fühlers (16) durch eine Öffnung (17) der Außenhülse (14) am Arbeitsende (11) aus der Außenhülse (14) bewegbar ist, wobei der Fühler (16) als länglicher, sich entlang der Längsachse (13) erstreckender Stift (16A) mit einem ersten, dem Anschlussende (9) zugewandten Ende (18A) und einem zweiten, dem Arbeitsende (11) zugewandten Ende (18B) ausgebildet ist, und
- einen elektromagnetischen Sensor (19) zur Detektion einer Relativbewegung zwischen der Außenhülse (14) und dem Fühler (16),
**dadurch gekennzeichnet, dass**
das erste, dem Anschlussende (9) zugewandte Ende (18A) des Fühlers (16) im Inneren der hohlen Außenhülse (14) des Bohrwerkzeugs (5, 6, 7, 8) aufgenommen ist, dass der elektromagnetische Sensor (19) entlang der Längsausdehnung (L) des Körpers (12) des Bohrwerkzeugs (5, 6, 7, 8) angeordnet ist und dass das Bohrwerkzeug (5, 6, 7, 8) einen sich entlang der Längsachse (13) erstreckenden Leitungskanal (20) für ein Behandlungsfluid aufweist.

2. Vorrichtung (1, 2, 3, 4) nach Anspruch 1, **dadurch gekennzeichnet, dass**
der elektromagnetische Sensor (19) einen induktiven Sensor (21) mit zumindest einer Spule (22) und einem Spulenkern (23), zum Beispiel einem hart- oder weichmagnetischen Magnetelement, insbesondere einem Ferritkörper (24), aufweist, wobei die zumindest eine Spule (22) und der Spulenkern (23) durch die Relativbewegung des Fühlers (16) zur Außenhülse (14) relativ zueinander bewegbar sind.

3. Vorrichtung (1, 2, 3, 4) nach Anspruch 2, **dadurch gekennzeichnet, dass**
der Spulenkern (23) mit dem Fühler (16) entlang der Längsachse (13) des Bohrwerkzeugs (5, 6, 7, 8) und relativ zur zumindest einen Spule (22) bewegbar ist.

4. Vorrichtung (1, 2, 3, 4) nach Anspruch 1, **dadurch gekennzeichnet, dass**
der elektromagnetische Sensor (19) einen Magnetsensor (25), insbesondere einen Hall-Sensor (26) oder einen Reed-Sensor, und zumindest ein Magnetelement (27) aufweist, wobei der Magnetsensor (25) und das zumindest eine Magnetelement (27) durch die Relativbewegung des Fühlers (16) zur Außenhülse (14) relativ zueinander bewegbar sind.

5. Vorrichtung (1, 2, 3, 4) nach Anspruch 1, **dadurch gekennzeichnet, dass**
der elektromagnetische Sensor (19) einen kapazitiven Sensor (28) mit zumindest zwei metallischen Elektroden (29, 30) aufweist, die einen Kondensator bilden, wobei eine Elektrode (30) durch die Relativbewegung des Fühlers (16) zur Außenhülse (14) relativ zu einer anderen Elektrode (29) bewegbar ist.

6. Vorrichtung (1, 2, 3, 4) nach Anspruch 5, **dadurch gekennzeichnet, dass**
der kapazitive Sensor (28) zumindest zwei im Wesentlichen plattenförmige Elektroden (29A, 29B) sowie eine mit dem Fühler (16) entlang der Längsachse (13) des Bohrwerkzeugs (5, 6, 7, 8) und relativ zu den zwei im Wesentlichen plattenförmigen Elektroden (29A, 29B) bewegbare Messelektrode (30) aufweist.

7. Vorrichtung (1, 2, 3, 4) nach einem der vorherstehenden Ansprüche, **dadurch gekennzeichnet, dass**
der Leitungskanal (20) für ein Behandlungsfluid eine Bohrung (31) in der Außenhülse (14) des Bohrwerkzeugs (5, 6, 7, 8) aufweist und / oder das den Fühler (16) vorspannende Federelement (15) durchsetzt.

8. Vorrichtung (1, 2, 3, 4) nach einem der vorherstehenden Ansprüche, **dadurch gekennzeichnet, dass**
der Leitungskanal (20) für ein Behandlungsfluid eine Bohrung (32) in dem Fühler (16) des Bohrwerkzeugs (5, 6, 7, 8) aufweist.

9. Vorrichtung (1, 2, 3, 4) nach einem der vorherstehenden Ansprüche, **dadurch gekennzeichnet, dass**
der Fühler (16) einen Führungsabschnitt (33A) aufweist, dessen Durchmesser in etwa der lichten Weite der hohlen Außenhülse (14) entspricht, so dass der Führungsabschnitt (33A) an der Innenwand der hohlen Außenhülse (14) gelagert ist, und dass der Fühler (16) einen zweiten Abschnitt (33B) aufweist, der durch einen Spalt (34), insbesondere einen Ringspalt, von der Innenwand der hohlen Außenhülse (14) beabstandet ist, wobei der Spalt (34) zumindest einen Teil des Leitungskanals (20) für ein Behandlungsfluid bildet.

10. Vorrichtung (1, 2, 3, 4) nach den Ansprüchen 8 und 9, **dadurch gekennzeichnet, dass**
die Bohrung (32) in dem Fühler (16), die den Leitungskanal (20) für ein Behandlungsfluid bildet, und der Spalt (34) durch eine Querbohrung (35) in dem Fühler (16) miteinander verbunden sind.

11. Vorrichtung (1, 2, 3, 4) nach einem der vorherstehenden Ansprüche, **dadurch gekennzeichnet, dass**
der Fühler (16) an seinem zweiten, dem Arbeitsende (11) zugewandten Ende (18B) eine exzentrisch zur Längsachse (13) angeordnete Fühlerspitze (36) aufweist.

12. Medizinische, insbesondere dentale, Behandlungsvorrichtung (37), umfassend eine Vorrichtung (1, 2, 3, 4) für den Schnellstopp eines medizinischen, insbesondere dentalen, Bohrwerkzeugs (5, 6, 7, 8) nach einem der vorherstehende Ansprüche, einen Antrieb (10) für das Bohrwerkzeug (5, 6, 7, 8), ein Handstück (38) zum Anschluss des Bohrwerkzeugs (5, 6, 7, 8) und eine Steuer- und / oder Regelvorrichtung (39) zum Empfang eines von dem elektromagnetischen Sensor (19) erzeugten Sensorsignals und zum Stoppen des Antriebs (10), wobei zumindest ein Teil des elektromagnetischen Sensors (19) am oder im Handstück (38) angeordnet ist, insbesondere an der Außenhülse (40) des Handstücks (38) befestigt ist.

13. Medizinische, insbesondere dentale, Behandlungsvorrichtung (37) nach Anspruch 12, **dadurch gekennzeichnet, dass**
der Sensor (19) im Bereich der Werkzeug-Einstecköffnung (41) des Handstücks (38) vorgesehen ist.

14. Medizinische, insbesondere dentale, Behandlungsvorrichtung (37) nach Anspruch 12 oder 13, **dadurch gekennzeichnet, dass**
ein erster Teil (19A) des Sensors (19) am Bohrwerkzeug (5, 6, 7, 8) vorgesehen ist und durch den Anschluss des Bohrwerkzeugs (5, 6, 7, 8) an das Handstück (38) lösbar mit dem Handstück (38) verbindbar ist und dass ein zweiter Teil (19B) des Sensors (19) über eine lösbare Verbindungsvorrichtung (42) direkt am Handstück (38) befestigbar ist, insbesondere an der Außenhülse (40) des Handstücks (38), so dass, wenn der erste Teil (19A) und der zweite Teil (19B) des Sensors (19) am Handstück (38) befestigt sind, der erste Teil (19A) und der zweite Teil (19B) des Sensors (19) operativ miteinander verbunden sind.

15. Medizinische, insbesondere dentale, Behandlungsvorrichtung (37) nach Anspruch 14, **dadurch gekennzeichnet, dass**
der erste Teil (19A) des Sensors (19) und / oder der zweite Teil (19B) des Sensors (19) an der Außenseite der Außenhülse (40) und / oder außerhalb der Außenhülse (40) des Handstücks (38) anordenbar sind.

## Claims

1. A device (1, 2, 3, 4) for the quick stop of a medical, in particular a dental, drilling tool (5, 6, 7, 8), comprising:
- a drilling tool (5, 6, 7, 8) having a connection end (9) for connection to a drive (10), an abrasive working end (11) for abrading material and having a body (12) which extends between the connection end (9) and the working end (11) and has a longitudinal extent (L) extending along a longitudinal axis (13) of the drilling tool (5, 6, 7, 8), wherein the drilling tool (5, 6, 7, 8) comprises a hollow outer sleeve (14), which holds a probe (16) that is prestressed by a spring element (15) and is movable along the longitudinal axis (13) in relation to the outer sleeve (14), so that at least a part of the probe (16) can be moved through an opening (17) of the outer sleeve (14) on the working end (11) out of the outer sleeve (14), wherein the probe (16) is designed as an elongated pin (16A), which extends along the longitudinal axis (13) and has a first end (18A) facing the connection end (9) and has a second end (18B) facing the working end (11), and
- an electromagnetic sensor (19) for detecting a relative movement between the outer sleeve (14) and the probe (16),
**characterized in that**
the first end (18A) of the probe (16) facing the connection end (9) is accommodated in the interior of the hollow outer sleeve (14) of the drilling tool (5, 6, 7, 8), that the electromagnetic sensor (19) is arranged along the longitudinal extent (L) of the body (12) of the drilling tool (5, 6, 7, 8) and that the drilling tool (5, 6, 7, 8) comprises a passage (20) for a treatment fluid extending along the longitudinal axis (13).

2. The device (1, 2, 3, 4) according to Claim 1, **characterized in that**
the electromagnetic sensor (19) comprises an inductive sensor (21) with at least one coil (22) and a coil core (23), for example, a hard or soft magnetic element, in particular a ferrite body (24), wherein the at least one coil (22) and the coil core (23) can be moved in relation to one another by the movement of the probe (16) in relation to the outer sleeve (14).

3. The device (1, 2, 3, 4) according to Claim 2, **characterized in that**
the coil core (23) is movable with the probe (16) along the longitudinal axis (13) of the drilling tool (5, 6, 7, 8) and in relation to the at least one coil (22).

4. The device (1, 2, 3, 4) according to Claim 1, **characterized in that**
the electromagnetic sensor (19) comprises a magnetic sensor (25), in particular a Hall sensor (26) or a Reed sensor, and at least one magnetic element (27), wherein the magnetic sensor (25) and the at least one magnetic element (27) can be moved in relation to one another by the movement of the probe (16) in relation to the outer sleeve (14).

5. The device (1, 2, 3, 4) according to Claim 1, **characterized in that**
the electromagnetic sensor (19) comprises a capacitive sensor (28) having at least two metallic electrodes (29, 30) forming a capacitor, wherein one electrode (30) can be moved in relation to another electrode (29) by the movement of the probe (16) in relation to the outer sleeve (14).

6. The device (1, 2, 3, 4) according to Claim 5, **characterized in that**
the capacitive sensor (28) comprises at least two substantially plate-shaped electrodes (29A, 29B) and one measuring electrode (30), which can be moved together with the probe (16) along the longitudinal axis (13) of the drilling tool (5, 6, 7, 8) and in relation to the two substantially plate-shaped electrodes (29A, 29B).

7. The device (1, 2, 3, 4) according to any one of the preceding claims, **characterized in that**
the passage (20) for a treatment fluid comprises a borehole (31) in the outer sleeve (14) of the drilling tool (5, 6, 7, 8) and / or that it passes through the spring element (15) prestressing the probe (16).

8. The device (1, 2, 3, 4) according to any one of the preceding claims, **characterized in that**
the passage (20) for a treatment fluid comprises a bore (32) in the probe (16) of the drilling tool (5, 6, 7, 8).

9. The device (1, 2, 3, 4) according to any one of the preceding claims, **characterized in that**
the probe (16) has a guide section (33A) whose diameter corresponds approximately to the inside diameter of the hollow outer sleeve (14), so that the guide section (33A) is supported on the inside wall of the hollow outer sleeve (14) and that the probe (16) has a second section (33B), which is spaced a distance apart from the inside wall of the hollow outer sleeve (14) by a gap (34), in particular an annular gap, wherein the gap (34) forms at least a part of the passage (20) for a treatment fluid.

10. The device (1, 2, 3, 4) according to Claims 8 and 9, **characterized in that**
the bore (32) in the probe (16), which forms the passage (20) for a treatment fluid, and the gap (34) are connected by a transverse borehole (35) in the probe (16).

11. The device (1, 2, 3, 4) according to any one of the preceding claims, **characterized in that**
the probe (16) comprises a probe tip (36) arranged eccentrically with the longitudinal axis (13) on its second end (18B) facing the working end (11).

12. A medical, in particular dental, treatment device (37), comprising a device (1, 2, 3, 4) for the quick stop of a medical, in particular dental, drilling tool (5, 6, 7, 8) according to any one of the preceding claims, a drive (10) for the drilling tool (5, 6, 7, 8), a handpiece (38) for connection of the drilling tool (5, 6, 7, 8) and a control and/or regulating device (39) for receiving a sensor signal generated by the electromagnetic sensor (19) and for stopping the drive (10), wherein at least one part of the electromagnetic sensor (19) is arranged in or on the handpiece (38), in particular on the outer sleeve (40) of the handpiece (38).

13. The medical, in particular dental, treatment device (37) according to Claim 12,
**characterized in that**
the sensor (19) is provided in the area of the tool insertion opening (41) of the handpiece (38).

14. The medical, in particular dental, treatment device (37) according Claim 12 or 13,
**characterized in that**
a first part (19A) of the sensor (19) is provided on the drilling tool (5, 6, 7, 8) and is detachably connectable to the handpiece (38) by means of the connection of the drilling tool (5, 6, 7, 8) to the handpiece (38), and that a second part (19B) of the sensor (19) can be attached directly to the handpiece (38) via a releasable connecting device (42), in particular being connectable to the outer sleeve (40) of the handpiece (38), so that when the first part (19A) and the second part (19B) of the sensor (19) are attached to the handpiece (38), the first part (19A) and the second part (19B) of the sensor (19) are operatively connected to one another.

15. The medical, in particular dental, treatment device (37) according Claim 14,
**characterized in that**
the first part (19A) of the sensor (19) and / or the second part (19B) of the sensor (19) can be arranged on the outside of the outer sleeve (40) and / or outside of the outer sleeve (40) on the handpiece (38).

## Revendications

1. Dispositif (1, 2, 3, 4) d'arrêt rapide d'un outil de forage médical, en particulier dentaire (5, 6, 7, 8), comprenant :
- un outil de forage (5, 6, 7, 8) comportant une extrémité de raccordement (9) pour la connexion à une entraînement (10), une extrémité opérationnelle abrasive (11) pour l'érosion de matière et un corps (12) s'étendant entre l'extrémité de raccordement (9) et l'extrémité opérationnelle (11) et ayant une extension longitudinale (L) s'étendant le long d'un axe longitudinal (13) de l'outil de forage (5, 6, 7, 8), l'outil de forage (5, 6, 7, 8) présentant un manchon extérieur creux (14) dans lequel est reçue une sonde (16) précontrainte par un élément à ressort (15) et qui peut bouger par rapport au manchon extérieur (14) le long de l'axe longitudinal (13) de manière à ce qu'au moins une partie de la sonde (16) puisse être sortie à travers une ouverture (17) du manchon extérieur (14) à l'extrémité opérationnelle (11) du manchon extérieur (14), la sonde (16) étant réalisée sous forme d'une tige allongée (16A) s'étendant le long de l'axe longitudinal (13) et dotée d'une première extrémité (18A) tournée vers l'extrémité de raccordement (9) et d'une seconde extrémité (18B) tournée vers l'extrémité opérationnelle (11) et
- un capteur électromagnétique (19) pour la détection d'un mouvement relatif entre le manchon extérieur (14) et la sonde (16),
**caractérisé en ce que**
la première extrémité (18A) tournée vers l'extrémité de raccordement (9) de la sonde (16) est reçue à l'intérieur du manchon extérieur creux (14) de l'outil de forage (5, 6, 7, 8), que le capteur électromagnétique (19) est disposé le long de l'extension longitudinale (L) du corps (12) de l'outil de forage (5, 6, 7, 8) et que l'outil de forage (5, 6, 7, 8) présente un canal conducteur (20) pour fluide de traitement s'étendant le long de l'axe longitudinal (13).

2. Dispositif (1, 2, 3, 4) selon la revendication 1, **caractérisé en ce que**
le capteur électromagnétique (19) présente un capteur électroinductif (21) comportant au moins une bobine (22) et un coeur de bobine (23), par exemple un élément magnétique dur ou doux, en particulier un corps ferritique (24), l'au moins une bobine (22) et le coeur de bobine (23) étant mobiles l'un par rapport à l'autre sous l'effet du mouvement relatif de la sonde (16) par rapport au manchon extérieur (14).

3. Dispositif (1, 2, 3, 4) selon la revendication 2, **caractérisé en ce que**
le coeur de bobine (23) est mobile avec la sonde (16) le long de l'axe longitudinal (13) de l'outil de forage (5, 6, 7, 8) et par rapport à au moins une bobine (22).

4. Dispositif (1, 2, 3, 4) selon la revendication 1, **caractérisé en ce que**
le capteur électromagnétique (19) présente un capteur magnétique (25), en particulier un capteur Hall (26) ou un capteur Reed, et au moins un élément magnétique (27), le capteur magnétique (25) et l'au moins un élément magnétique (27) étant mobiles l'un par rapport à l'autre sous l'effet du mouvement relatif de la sonde (16) par rapport au manchon extérieur (14).

5. Dispositif (1, 2, 3, 4) selon la revendication 1, **caractérisé en ce que**
le capteur électromagnétique (19) présente un capteur capacitif (28) comportant au moins deux électrodes métalliques (29, 30) qui forment un condensateur, une électrode (30) étant mobile par rapport à l'autre électrode (29) sous l'effet du mouvement relatif de la sonde (16) par rapport au manchon extérieur (14).

6. Dispositif (1, 2, 3, 4) selon la revendication 5, **caractérisé en ce que**
le capteur capacitif (28) présente au moins deux électrodes (29A, 29B) sensiblement en forme de plaques et une électrode de mesure (30) mobile avec la sonde (16) le long de l'axe longitudinal (13) de l'outil de forage (5, 6, 7, 8) et mobile par rapport aux deux électrodes (29A, 29B) sensiblement en forme de plaques (29A, 29B).

7. Dispositif (1, 2, 3, 4) selon une des revendications précédentes, **caractérisé en ce que**
le canal conducteur (20) pour fluide de traitement présente un alésage (31) pratiqué dans le manchon extérieur (14) de l'outil de forage (5, 6, 7, 8) et / ou dans l'élément à ressort (15) précontraignant la sonde (16).

8. Dispositif (1, 2, 3, 4) selon une des revendications précédentes, **caractérisé en ce que**
le canal conducteur (20) pour fluide de traitement présente un alésage (32) pratiqué dans la sonde (16) de l'outil de forage (5, 6, 7, 8).

9. Dispositif (1, 2, 3, 4) selon une des revendications précédentes, **caractérisé en ce que**
la sonde (16) présente une section de guidage (33A) dont le diamètre équivaut approximativement au diamètre intérieur du manchon extérieur creux (14) de manière à ce que la section de guidage (33A) s'appuie sur la paroi intérieure du manchon extérieur creux (14) et que la sonde (16) présente une seconde section (33B) qui est espacée par un intervalle (34), en particulier un intervalle annulaire, de la paroi intérieure du manchon extérieur creux (14), l'intervalle (34) formant au moins une partie du canal conducteur (20) pour fluide de traitement.

10. Dispositif (1, 2, 3, 4) selon les revendications 8 et 9, **caractérisé en ce que**
l'alésage (32) pratiqué dans la sonde (16) qui forme le canal conducteur (20) pour fluide de traitement et l'intervalle (34) sont reliés ensemble par un alésage transversal (35) pratiqué dans la sonde (16).

11. Dispositif (1, 2, 3, 4) selon une des revendications précédentes, **caractérisé en ce que**
la sonde (16) présente une pointe de palpage (36) disposée excentriquement par rapport à l'axe longitudinal (13) à sa seconde extrémité (18B) tournée vers l'extrémité opérationnelle (11).

12. Dispositif de traitement médical, en particulier dentaire (37), comprenant un dispositif (1, 2, 3, 4) d'arrêt rapide d'un outil de forage médical, en particulier dentaire (5, 6, 7, 8), selon une des revendications précédentes, une commande (10) pour l'outil de forage (5, 6, 7, 8), une pièce manuelle (38) pour le raccordement de l'outil de forage (5, 6, 7, 8) et un dispositif de commande et / ou de réglage (39) pour la réception d'un signal de détection généré par le capteur électromagnétique (19) et pour l'arrêt de la commande (10), au moins une partie du capteur électromagnétique (19) étant disposée sur ou dans la pièce manuelle (38), en particulier fixée au manchon extérieur (40) de la pièce manuelle (38).

13. Dispositif de traitement médical, en particulier dentaire (37), selon la revendication 12, **caractérisé en ce que**
le capteur (19) est prévu au niveau de l'orifice de fichage d'outil (41) de la pièce manuelle (38).

14. Dispositif de traitement médical, en particulier dentaire (37), selon la revendication 12 ou 13, **caractérisé en ce**
**qu'**une première partie (19A) du capteur (19) est prévue sur l'outil de forage (5, 6, 7, 8) et peut être reliée de manière dissociable à la pièce manuelle (38) par la connexion de l'outil de forage (5, 6, 7, 8) à la pièce manuelle (38) et qu'une seconde partie (19B) du capteur (19) peut être fixée par un dispositif de connexion dissociable (42) directement à la pièce manuelle (38), en particulier au manchon extérieur (40) de la pièce manuelle (38), de manière à ce que, lorsque la première partie (19A) et la seconde partie (19B) du capteur (19) sont fixées à la pièce manuelle (38), la première partie (19A) et la seconde partie (19B) du capteur (19) soient reliées opérationnellement entre elles.

15. Dispositif de traitement médical, en particulier dentaire (37) selon la revendication 14, **caractérisé en ce que**
la première partie (19A) du capteur (19) et / ou la seconde partie (19B) du capteur (19) peuvent être disposées sur l'extérieur du manchon extérieur (40) et / ou en dehors du manchon extérieur (40) de la pièce manuelle (38).
